# EUROPÄISCHE PATENTSCHRIFT

(11) **EP 2 732 068 B1**
(45) Veröffentlichungstag und Bekanntmachung des Hinweises auf die Patenterteilung: **04.03.2015**
(21) Anmeldenummer: 12738397.4
(22) Anmeldetag: 10.07.2012
(51) Int. Cl.: C23C 14/08, C23C 14/58, C23C 14/00, A61B 1/00, A61L 31/08

(54) **VERFAHREN ZUR VERBESSERUNG DER VERSCHLEISSBESTÄNDIGKEIT VON EINGEFÄRBTEN CHIRURGISCHEN INSTRUMENTEN**
METHOD FOR IMPROVING THE WEAR RESISTANCE OF DYED SURGICAL INSTRUMENTS
PROCÉDÉ POUR AMÉLIORER LA RÉSISTANCE À L'USURE D'INSTRUMENTS CHIRURGICAUX COLORÉS

(30) Priorität: 15.07.2011 DE 102011107787
(43) Veröffentlichungstag der Anmeldung: 21.05.2014
(73) Patentinhaber: Oerlikon Trading AG, Trübbach, 9477 Trübbach (CH)
(72) Erfinder: JANSSEN, Albert Peter Gerhard, CH-7000 Chur (CH); SINICCO, Gabriella, CH - 1007 Lausanne (CH)
(74) Vertreter: Kempkens, Anke
(86) Internationale Anmeldenummer: PCT/EP2012/002910
(87) Internationale Veröffentlichungsnummer: WO 2013/010647

(56) Entgegenhaltungen:
- EP-A1- 0 608 997
- EP-A1- 1 652 963
- DE-C1- 19 809 932
- US-A1- 2010 255 337
- GEETHA M ET AL: "Ti based biomaterials, the ultimate choice for orthopaedic implants - A review", PROGRESS IN MATERIALS SCIENCE, PERGAMON PRESS, GB, Bd. 54, Nr. 3, 1. Mai 2009 (2009-05-01), Seiten 397-425, XP025947704, ISSN: 0079-6425, DOI: 10.1016/J.PMATSCI.2008.06.004 [gefunden am 2008-12-11]

## Beschreibung

Die vorliegende Erfindung betrifft ein Verfahren zur Verbesserung der Verschleissbeständigkeit von eingefärbten bzw. einzufärbenden chirurgischen Instrumenten.

Farbige chirurgische Instrumente sind sehr gebräuchlich. Die Farbgebung erlaubt beispielsweise eine klare Zuordnung zwischen einem Instrument und einem Implantat. Aber auch zwischen unterschiedlichen Instrumenten kann besser unterschieden werden, wenn diese eine unterschiedliche Farbe besitzen. Hauptzweck der Farbgebung , welche in der Regel durch Farbanodisierung erreicht wird, ist die Identifizierung von Implantaten vor und während des operativen Eingriffs (farbige Größenkennzeichnung). Die Anwendungen umfassen u.a. sowohl dentale- als auch orthopädische Implantate sowie Osteosyntheseprodukte wie Schrauben, Platten oder Instrumente. Als Nebeneffekt bewirkt die Einfärbung ebenfalls die Hemmung der Freisetzung von Aluminium- und Vanadium-Ionen aus der Titanlegierung.

Aus der DE 198 09 932 ist ein Verfahren zum Einfärben von chirurgischen Instrumenten, die aus vakuumbeständigen, hochtemperaturfestem Kunststoff oder aus Stahl bestehen, bekannt. Die Instrumente werden demgemäss mit einer dünnen Titanschicht mit einer Dicke zwischen 2µm und 10µm in einer Vakuumanlage bekannter Art beschichtet. Anschliessend wird diese Beschichtung durch anodische Oxidation an ihrer Oberfläche in einer Schichtdicke im Nanometerbereich oxidiert. Hierzu wird das Instrument in einen Elektrolyten gehalten, der beispielsweise 1.8% Zitronensäure enthält und dass zu oxidierende Teil wird als Anode geschaltet. Es wird beschrieben, dass ein titanblech als Kathode dienen kann. In der DE 198 09 932 wird dann eine Gleichspannung von maximal 130 Volt an die Elektroden angelegt, wobei die Grösse der Gleichspannung Einfluss auf die erzielbare Farbe hat. Mit einer Stromdichte in der Grössenordnung zwischen 100 und 200mA wird dann die Titanschicht oberflächlich anodisch oxidiert, und zwar mit einer Schichtdicke in der Grössenordnung von 50nm und 250nm.

Gegen Verschleiss sind die oben genannten chirurgischen Instrumente jedoch bisher nicht geschützt, da die mit den Instrumenten zu bearbeitenden organischen Materialien meist weich sind und Implantaten meistens bereits bei ihrer Herstellung ihre fertige Form verliehen wird. Trotzdem zeigen eingefärbte chirurgische Instrumente gemäss Stand der Technik bereits nach kurzer Zeit fleckenhafte Farbveränderungen welche deren Gebrauch zumindest beim Patienten Zweifel aufkommen lässt.

Der Erfindung liegt daher die Aufgabe zugrunde ein Verfahren anzugeben, mit dem die oben beschriebenen Abnutzungserscheinungen vermieden oder zumindest zeitlich hinausgezögert werden können.

Erfindungsgemäss wird diese Aufgabe durch das Verfahren gemäss Anspruch 1 und ein chirurgisches Instrument gemäss Anspruch 11 gelöst. In den Unteransprüchen sind unterschiedliche und bevorzugte Ausführungsformen beschrieben.

Zu ihrem Erstaunen konnten die Erfinder herausfinden, dass es sich bei den Fleckenerscheinungen um Verschleisserscheinungen handelt. Wahrscheinlich kommt es im chirurgisch hektischen Alltag häufiger vor als bisher vermutet, dass Instrumente anecken an z.B. Behältnisse, aus denen sie herausgenommen oder in die sie hineingelegt werden, anschlagen. Manchmal kommt es auch vor, dass ein solches Instrument fallen gelassen wird und durch den Sturz einen erheblichen Stoss erhält.

Verleiht der eine Titanoxidschicht tragende Untergrund dieser Titanoxidschicht nicht genügend Stabilität, so bricht diese ein und es können die oben beschriebenen Flecken entstehen. Insbesondere ist eine wie in der DE 198 09 932 beschriebene dünne Titanschicht nicht in der Lage der durch Anodisierung entstandenen Titanoxidschicht genügen Stabilität zu vermitteln. Demgegenüber haben die Massnahmen der vorliegenden Erfindung eine stabilisierende Wirkung. Indem nämlich zwischen dem Grundkörper und der Titanoxidschicht eine Hartstoffschicht vorgesehen ist, erhält die Unterlage der Titanoxidschicht ausreichend Stabilität um der Titanoxidschicht genügend Halt zu verleihen.

Falls unterhalb der Titanoxidschicht eine metallischer Titanschicht vorgesehen ist, etwa um durch anodische Oxidation die Titanoxidschicht erzeugen zu können, wird die Hartstoffschicht zwischen Substrat und metallischer Titanschicht vorgesehen. Bei dieser metallischen Titanschicht sollte es sich allerdings um eine dünne Schicht handeln.

Als "dünn" im Sinne der vorliegenden Erfindung gilt eine Schicht, deren Dicke nicht 20µm überschreitet. Bei Titanschichtdicken grösser als 20µm wirken sich die relativ geringe Härte von Titan negativ aus, so dass die stabilisierende Haftschicht für die Titanoxidschicht ihre Wirkung verliert.

Manche medizinischen Anwendungen schliessen allerdings die Verwendung von metallischen Titanschichten aus. Zur Herstellung der Titanoxidschicht ist dann die Anwendung der anodischen Oxidation keine Option. Es gibt aber die Möglichkeit eine solche Titanoxidschicht direkt durch ein Vakuumbeschichtungs-verfahren, insbesondere ein PVD-Verfahren zu realisieren. Besonders geeignet ist das als reaktives Zerstäuben bekannte PVD-Verfahren, bei dem von einem Titantarget mittels lonenbeschuss Material herausgeschlagen wird, welches mit dem Reaktivgas reagiert. Vorliegend umfasst das Reaktivgas Sauerstoff, so dass sich auf dem zu beschichtenden Substrat die erwünschte Titanoxidschicht bildet. Der Vorteil eines solchen Vorgehens liegt u.a. darin, dass es nicht nötig ist, eine metallische Titanschicht zwischen Substrat und Oxidschicht vorzusehen.

Selbst wenn eine metallische Titanschicht erwünscht ist, kann das Zerstäubungsverfahren zur Erzeugung der Titanoxidschicht mit Vorteil eingesetzt werden, da es ohne weiteres möglich ist, das Zerstäubungsverfahren zunächst ohne Reaktivgas zu betreiben, so dass sich zunächst eine metallische Titanschicht auf dem Substrat ablegt.

Die nachfolgende Beschreibung einer bevorzugten Ausführungsform dient der näheren Erläuterung der Erfindung. Als Beispiel wird eine erfindungsgemäss behandelte chirurgische Zange beschrieben. Es ist dabei von Vorteil, die Oberfläche des Zangenkörpers zunächst zu reinigen, da die Schicht-Substrat-Grenzfläche zur Sicherstellung der Adhäsion möglichst frei von Kontaminationen sein sollte. Wird zum Beispiel Stahl verwendet, so bildet sich häufig eine Chromoxidschicht an der Stahloberfläche welche vor der Vakuumbeschichtung entfernt werden sollte. Dies kann durch geeignete Verfahren, wie zum Beispiel DC- oder RF-Sputterreinigung in einem Argon-Wasserstoff-Plasma gemacht werden. Die Chirurgenzange wird dann in einer Vakuumbeschichtungsanlage mittels Beschichtung aus der Gasphase (PVD= physical vapor deposition) mit zuerst einer 0.5µm dicken Chromschicht als Haftschicht beschichtet. In derselben Beschichtungsanlage und ohne das Vakuum zu brechen wird anschliessend eine 3µm dicke Chromnitridschicht mittels Zerstäuben von einem Chrom Margnetrontarget und unter Einlassung von Stickstoff in die Kammer als Reaktivgas aufgebracht. Ebenfalls in derselben Beschichtungsanlage und ohne das Vakuum zu Brechen wird anschliessend von einen Titantarget zerstäubt und dementsprechend eine 5µm dicke Titanschicht aufgebracht.

Die derart beschichtete Zange wird anschliessend und analog zu dem oben im Rahmen der DE 198 09 932 beschriebenen Verfahren einer anodischen Oxidation unterworfen. Es sind Oxidschichtdicken von zwischen 10nm und 250nm sinnvoll. Die Zange besitzt die vom Verwender gewünschte Farbe. Aufgrund der unter der Titanschicht liegenden Hartstoffschicht bleibt die Farbe auch dann lokal erhalten, wenn diese den Stössen wie sie im chirurgischen Alltag vorkommen, ausgesetzt ist.

Gemäss einer zweiten Ausführungsform wird anstelle der Chromnitridschicht eine Titannitridschicht mittels Lichtbogenverdampfen aufgebracht. Es ist dann möglich, im Beschichtungsprozess den Stickstoffzufluss kontinuierlich und/oder Schrittweise zu ändern, insbesondere zu verringern, wodurch eine Gradientenschicht erzeugt werden, an deren Ende kein Stickstoff mehr vorhanden ist. Hierdurch entsteht ein guter Übergang zur Titanschicht.

Es kann auch bei der Verwendung von CrN ein Gradient realisiert und damit ein Übergang zum Titan realisiert werden. Hierzu ist es aber dann in der Regel erforderlich, zwei Targets parallel laufen zu lassen(Cr und Ti) und die Sputterleistung des Cr parallel zum Stickstofffluss zu reduzieren. Dies ist zwar machbar, technisch jedoch etwas aufwändiger.

Gemäss einer weiteren Ausführungsform der vorliegenden Erfindung wird, wie oben beschrieben, eine Chrom-Haftschicht und eine Chromnitrid-Hartstoffschicht auf den Zangenkörper aufgebracht. Anschliessend wird von einem Titantarget zerstäubt und bei Zugabe von Reaktivgas, welches Sauerstoff umfasst und vorzugsweise im Wesentlichen aus Sauerstoff besteht, auf dem Zangenkörper eine Titanoxidschicht abgeschieden.

Es sei bemerkt, dass falls das Substratmaterial eine genügend hohe Eigenhärte aufweist und das Titanoxid auf dem Substrat in ausreichender Weise haftet, eventuell auf die Hartstoffschicht verzichtet werden kann.

Es wurde Verfahren offenbart zur Verbesserung der Verschleissbeständigkeit von eingefärbten chirurgischen Instrumenten, die ein vakuumbeständiges Material als Grundkörper besitzen, welcher im Rahmen eines Vakuumbeschichtungsschrittes mit einer dünnen Titanschicht überzogen wird und die beschichtete Oberfläche einer anodischen Oxidation unterzogen wird. Alternativ ist es möglich, die Titanoxidschicht mittels reaktiven Abscheidens aus der Gasphase in einer Vakuumbschichtungsanlage herzustellen, so dass auf die anodische Oxidation und eventuell auch auf die Titanschicht verzichtet werden kann. Das Verfahren ist dadurch gekennzeichnet, dass im Rahmen des Vakuumbeschichtungsschrittes vorgängig des Überzugs mit der Titanoxidschicht zumindest Teile des Grundkörpers mit einer den Verschleissschutz verbessernden Hartstoffschicht beschichtet werden.

Die Hartstoffschicht kann mittels eines Nitrids und/oder Oxides von zumindest einem Metall und/oder von zumindest einer Legierung gebildet werden.

Das Nitrid und/oder Oxid kann ein mit Titan und/oder Chrom gebildetes sein.

Die Hartstoffschicht kann mittels einer Chromnitridschicht vorzugsweise mittels Magnetronsputtern gebildet werden.

Die Hartstoffschicht kann mittels einer Titannitridschicht vorzugsweise mittels Lichtbogenverdampfen gebildet werden.

Die Hartstoffschicht kann mit mindestens 1.5µm und maximal 20µm Dicke aufgebracht werden und wird bevorzugt zwischen 2µm und 5µm aufgebracht.

Die Titanschicht wird mindestens 1µm dick, bevorzugt zwischen 2µm und 10µm dick und vorzugsweise direkt auf die Hartstoffschicht aufgebracht.

Zur Verbesserung der Haftung der Hartstoffschicht auf dem Grundkörper kann zwischen Grundkörper und Hartstoffschicht eine Haftschicht vorzugsweise aus Chrom vorgesehen sein.

Es wurde ein chirurgisches Instrument mit einem Grundkörper und einer Titan-Beschichtung offenbart, dessen äusserste Schicht eine Oxidschicht umfasst wobei zwischen dem Grundkörper und der Titan-Beschichtung eine Hartstoffschicht vorgesehen ist.

Die Hartstoffschicht kann vorzugsweise zwischen 2µm und 10µm dick sein und Titannitrit und/oder Chromnitrid umfassen.

Zwischen dem Grundkörper und der Hartstoffschicht kann eine Haftschicht, vorzugsweise eine Chromschicht, vorgesehen sein.

Die vorliegende Erfindung wurde beispielhaft anhand einer farbgebenden Titanoxidschicht beschrieben. Zwar ist eine solche Schicht zu bevorzugen, allerdings lassen sich ähnliche Resultate durch Zirkonoxid (z.B. ZrO) und/oder Nioboxid (z.B. Nb₂O) erreichen. Ausserdem ist es möglich ein Wechselschichtsystem (mit Titanoxid und/oder Zirkonoxid und/oder Nioboxid) aufzubauen, welches auf der Basis von Interferenzwirkung der Schichten zu besonderen Farbeindrücken führen kann.

## Patentansprüche

1. Verfahren zur Verbesserung der Verschleissbeständigkeit von eingefärbten chirurgischen Instrumenten die ein vakuumbeständiges Material als Grundkörper besitzen, welcher mit einer Titanoxidschicht überzogen wird, **dadurch gekennzeichnet dass** im Rahmen eines Vakuumbeschichtungsschrittes vorgängig des Überzugs mit der Titanoxidschicht zumindest Teile des Grundkörpers mit einer den Verschleissschutz verbessernden Hartstoffschicht beschichtet werden.

2. Verfahren nach Anspruch 1, **dadurch gekennzeichnet, dass** die Herstellung der Titanoxidschicht eine Abscheidung aus der Gasphase in einer Vakuumbeschichtungsanlage umfasst, wobei im Zuge dieser Abscheidung entweder Titanoxid reaktiv mittels zerstäubtem oder verdampftem Titan und unter Zuhilfenahme von Sauerstoff als Reaktivgas abgeschieden wird oder im Rahmen der Gasphasenabscheidung eine dünne Titanschicht auf den Grundkörper abgeschieden wird und die mit Titan beschichtete Oberfläche eine anodischen Oxidation unterzogen wird.

3. Verfahren nach Anspruch 1 oder 2, **dadurch gekennzeichnet, dass** die Hartstoffschicht mittels eines Nitrides und/oder Oxides von zumindest einem Metall und/oder von zumindest einer Legierungen gebildet wird.

4. Verfahren nach Anspruch 2, **dadurch gekennzeichnet, dass** das Nitrid und/oder Oxid ein mit Titan und/oder Chrom gebildetes ist.

5. Verfahren nach einem der vorangehenden Ansprüche, **dadurch gekennzeichnet, dass** die Hartstoffschicht mittels einer Chromnitridschicht gebildet wird

6. Verfahren nach einem der Ansprüche 1 bis 3, **dadurch gekennzeichnet, dass** die Hartstoffschicht mittels einer Titannitridschicht gebildet wird.

7. Verfahren nach einem der vorangehenden Ansprüche, **dadurch gekennzeichnet, dass** die Hartstoffschicht mindestens 1.5µm und maximal 20µm dick aufgebracht wird.

8. Verfahren nach einem der vorangehenden Ansprüche, **dadurch gekennzeichnet, dass** im Falle einer abgeschiedenen Titanschicht diese mindestens 1µm dick, bevorzugt zwischen 2µm und 10µm dick.

9. Verfahren nach einem der vorangehenden Ansprüche, **dadurch gekennzeichnet, dass** zur Verbesserung der Haftung der Hartstoffschicht auf dem Grundkörper zwischen Grundkörper und Hartstoffschicht eine Haftschicht vorzugsweise aus Chrom vorgesehen ist.

10. Verfahren nach einem der vorangehenden Ansprüche, **dadurch gekennzeichnet, dass** die Hartstoffschicht als Gradientenschicht aufgebracht wird und hierdurch ein gradueller Übergang zur Titanoxidschicht oder zur Titanschicht realisiert wird.

11. Chirurgisches Instrument mit einem Grundkörper und einer Titanoxid-Beschichtung **dadurch gekennzeichnet, dass** zwischen dem Grundkörper und der Titanoxid-Beschichtung eine Hartstoffschicht vorgesehen ist.

12. Chirurgisches Instrument nach Anspruch 11, **dadurch gekennzeichnet, dass** die Hartstoffschicht zwischen 2µm und 10µm dick ist.

13. Chirurgisches Instrument nach einem der Ansprüche 11 und 12, **dadurch gekennzeichnet, dass** die Hartstoffschicht Titannitrit und/oder "Chromnitrid umfasst.

14. Chirurgisches Instrument nach einem der Ansprüche 11 bis 13 **dadurch gekennzeichnet, dass** zwischen dem Grundkörper und der Hartstoffschicht eine Haftschicht vorgesehen ist.

15. Chirurgisches Instrument nach einem der Ansprüche 11 bis 14, **dadurch gekennzeichnet, dass** die Hartstoffschicht als Gradientenschicht ausgestaltet ist und ein gradueller Übergang zur Titanoxidschicht realisiert ist.

## Claims

1. Method for improving the wear resistance of dyed surgical instruments having a vacuum-resistant material as the main body which is coated with a thin titanium oxide layer, **characterized in that**, over the course of the vacuum coating step, prior to coating with the titanium oxide layer, at least parts of the main body are coated with a layer of hard material which improves the wear protection.

2. Method according to claim 1, **characterized in that** the production of the titanium oxide layer comprises a deposition from the vapor phase in a vacuum coating facility, wherein in the course of this deposition either titanium oxide is deposited reactively by means of sputtered or vaporized titanium and with the aid of oxygen as reactive gas or in the course of the vapor deposition a thin titanium layer is deposited on the main body and the surface coated with titanium is subjected to anodic oxidation.

3. Method according to claim 1 or 2, **characterized in that** the layer of hard material is formed by means of a nitride and/or oxide of at least one metal and/or of at least one alloy.

4. Method according to claim 2, **characterized in that** the nitride and/or oxide is formed with titanium and/or chromium.

5. Method according to one of the preceding claims, **characterized in that** the layer of hard material is formed by means of a chromium nitride layer.

6. Method according to one of the claims 1 to 3, **characterized in that** the layer of hard material is formed by means of a titanium nitride layer.

7. Method according to one of the preceding claims, **characterized in that** the layer of hard material is deposited with a thickness of at least 1.5µm and maximum 20µm.

8. Method according to one of the preceding claims, **characterized in that** in the case of a deposited titanium layer, the latter is deposited with a thickness of at least 1µm, preferably between 2µm and 10µm.

9. Method according to one of the preceding claims, **characterized in that** to improve the adhesion of the layer of hard material onto the main body, a bonding layer, preferably of chromium, is provided between the main body and the layer of hard material.

10. Method according to one of the preceding claims, **characterized in that** the layer of hard material is deposited as a gradient layer and thus a gradual transition to the titanium oxide layer or to the titanium layer is achieved.

11. Surgical instrument with a main body and a titanium oxide coating, **characterized in that** a layer of hard material is provided between the main body and the titanium oxide coating.

12. Surgical instrument according to claim 11, **characterized in that** the layer of hard material has a thickness between 2µm and 10µm.

13. Surgical instrument according to one of the claims 11 and 12, **characterized in that** the layer of hard material comprises titanium nitride and/or chromium nitride.

14. Surgical instrument according to one of the claims 11 to 13, **characterized in that** a bonding layer is provided between the main body and the layer of hard material.

15. Surgical instrument according to one of the claims 11 to 14, **characterized in that** the layer of hard material is formed as a gradient layer and thus a gradual transition to the titanium oxide layer is achieved.

## Revendications

1. Procédé pour améliorer la résistance à l'usure d'instruments chirurgicaux colorés qui ont un matériau résistant au vide comme corps de base, qui est revêtu d'une couche d'oxyde de titane, **caractérisé par le fait que** dans le cadre d'une étape de revêtement sous vide, avant le revêtement avec la couche d'oxyde de titane, au moins des parties du corps de base sont revêtues avec une couche de matière dure améliorant la protection contre l'usure.

2. Procédé selon la revendication 1 **caractérisé par le fait que** la fabrication de la couche d'oxyde de titane comprend un dépôt à partir d'une phase gazeuse dans une installation de revêtement sous vide, dans lequel, au cours de ce dépôt, soit de l'oxyde de titane est déposé au moyen de titane atomisé ou vaporisé dans une façon réactive et par l'aide de l'oxygène comme gaz réactif, soit une couche mince de titane est déposé sur le corps de base dans le cadre du dépôt en phase gazeuse et la surface revêtue de titane est soumise à une oxydation anodique.

3. Procédé selon la revendication 1 ou 2 **caractérisé par le fait que** la couche de matière dure est formée au moyen d'un nitrure et/ou oxyde de pour le moins un métal et/ou de pour le moins un alliage.

4. Procédé selon la revendication 2 **caractérisé par le fait que** le nitrure et/ou l'oxyde est l'un formé avec le titane et/ou le chrome.

5. Procédé selon l'une des revendications précédentes, **caractérisé par le fait que** la couche de matière dure est formée au moyen d'une couche de nitrure de chrome.

6. Procédé selon l'une des revendications 1 à 3, **caractérisé par le fait que** la couche de matière dure est formée au moyen d'une couche de nitrure de titane.

7. Procédé selon l'une des revendications précédentes, **caractérisé par le fait que** la couche de matière dure est appliquée avec une épaisseur de pour le moins 1,5 µm et de 20 µm au maximum.

8. Procédé selon l'une des revendications précédentes, **caractérisé par le fait que** dans le cas d'une couche de titane déposée celle-ci a une épaisseur de pour le moins 1 µm, préférablement entre 2 µm et 10 µm.

9. Procédé selon l'une des revendications précédentes, **caractérisé par le fait que** pour l'amélioration de l'adhésion de la couche de matière dure sur le corps de base entre le corps de base et la couche de matière dure une couche adhésive préférablement de chrome est prévue.

10. Procédé selon l'une des revendications précédentes, **caractérisé par le fait que** la couche de matière dure est appliquée comme couche gradient en réalisant de ce fait une transition graduelle vers la couche d'oxyde de titane ou la couche de titane.

11. Instrument chirurgical ayant une corps de base et une revêtement d'oxyde de titane **caractérisé par le fait qu'**entre le corps de base et le revêtement d'oxyde de titane une couche de matière dure est prévue.

12. Instrument chirurgical selon la revendication 11 **caractérisé par le fait que** la couche de matière dure a une épaisseur entre 2 µm et 10 µm.

13. Instrument chirurgical selon l'une des revendications 11 et 12 **caractérisé par le fait que** la couche de matière dure comprend le nitrure de titane et/ou le nitrure de chrome.

14. Instrument chirurgical selon l'une des revendications 11 à 13, **caractérisé par le fait qu'**entre le corps de base et la couche de matière dure une couche adhésive est prévue.

15. Instrument chirurgical selon l'une des revendications 11 à 14, **caractérisé par le fait que** la couche de matière dure est configurée en tant que couche gradient et une transition graduelle vers la couche d'oxyde de titane est réalisée.
